# FASCICULE DE BREVET EUROPEEN

(11) **EP 4 444 374 B1**
(45) Date de publication et mention de la délivrance du brevet: **03.12.2025**
(21) Numéro de dépôt: 22802676.1
(22) Date de dépôt: 24.10.2022
(51) Int. Cl.: A61M 1/00, A61K 35/35, C12M 1/00

(54) **PROCEDE DE PURIFICATION D'UN TISSU ADIPEUX ET TISSU ADIPEUX PURIFIE ASSOCIE**
VERFAHREN ZUR REINIGUNG VON FETTGEWEBE UND ZUGEHÖRIGES GEREINIGTES FETTGEWEBE
METHOD FOR PURIFYING ADIPOSE TISSUE AND ASSOCIATED PURIFIED ADIPOSE TISSUE

(30) Priorité: 08.12.2021 FR 2113138
(43) Date de publication de la demande: 16.10.2024
(73) Titulaire: Neosyad, 13290 Aix-en-Provence (FR)
(72) Inventeur: ROCHE, Régis, 83510 LORGUES (FR); CABAUD, François, 13510 EGUILLES (FR); NELISSEN, Xavier, 4453 VILLERS SAINT SIMEON (BE)
(74) Mandataire: Cabinet Beau de Loménie
(86) Numéro de dépôt international: PCT/FR2022/052008
(87) Numéro de publication internationale: WO 2023/105127

(56) Documents cités:
- US-A1- 2013 087 643
- US-A1- 2018 221 550
- US-A1- 2020 061 259

## Description

### Domaine Technique

La présente invention concerne un procédé de purification d'un tissu adipeux et un tissu adipeux purifié associé adapté à une réintroduction dans le corps d'un patient. L'invention trouve notamment une application pour les opérations de greffe autologue de tissu adipeux (désignées par « lipofilling » en langue anglaise) à visées esthétiques et reconstructrices. L'invention trouve, en particulier, une utilisation pour les opérations de chirurgie mammaire, la mise en œuvre de l'invention n'étant toutefois pas limitée à cette application.

### Technique antérieure

Des opérations de greffe autologue de tissu adipeux sont utilisées en chirurgie notamment en chirurgie mammaire afin de modeler le sein pour lui donner un aspect plus naturel après une perforante de l'artère épigastrique profonde intérieure (« Deep Inferior Epigastric Perforator », DIEP), une reconstruction par lambeau de grand dorsal, ou après le placement d'un implant mammaire. Ces opérations consistent à prélever de la graisse d'un patient dans une zone donneuse pour la réintroduire ensuite dans la zone d'intérêt du corps du patient. Ces opérations n'ont actuellement pas un rendu reproductible et peuvent nécessiter plusieurs interventions du fait de la résorption de la matière introduite (perte de volume rencontrée après l'opération). En outre, la préparation de la graisse en vue de sa réintroduction est réalisée par un opérateur, chirurgien ou assistant du chirurgien, ce qui engendre un coût de mise en œuvre de l'opération relativement élevé et un risque d'erreur ou de perte en reproductibilité. Il est donc souhaitable de proposer une technique qui simplifie et rende plus fiables les opérations existantes.

Le document US 2020/0054824 a cherché à proposer une amélioration par mise en œuvre d'un système en boucle fermée dans lequel la graisse est récupérée pour être préparée en vue de sa réintroduction en étant mélangée à un liquide de lavage dans un dispositif de filtration par gravité commercialisé sous la référence commerciale Revolve^{®}, et être ensuite réintroduite dans le corps du patient à l'aide d'une pompe centrifuge. Il est toutefois possible d'améliorer la qualité de la graisse réintroduite. Un autre exemple d'art antérieur est décrit dans US2020/061259 A1.

### Exposé de l'invention

La présente invention vise à surmonter les inconvénients de l'art antérieur et concerne un procédé de purification d'un tissu adipeux, comprenant au moins :
- l'ajout d'un liquide de lavage à un tissu adipeux à purifier comprenant des matières polluantes et présent dans un volume de traitement délimité par des moyens de rétention du tissu adipeux et de filtration des matières polluantes,
- la filtration d'une fraction des matières polluantes du tissu adipeux, contenue dans le liquide de lavage, par centrifugation du contenu du volume de traitement par mise en rotation des moyens de rétention et de filtration, et
- une évacuation du liquide de lavage contenant la fraction des matières polluantes filtrée.

De manière caractéristique, l'invention met en œuvre une purification du tissu adipeux par filtration des matières polluantes au travers des moyens de rétention et de filtration du fait de la mise en rotation de ces derniers. Cela permet avantageusement d'optimiser la qualité du tissu adipeux purifié en réduisant la présence de liquide interstitiel et en s'affranchissant de la présence d'un organe de mélange ou de malaxage qui risquerait d'affecter l'intégrité des cellules adipeuses comme dans le cas du dispositif commercialisé sous la référence commerciale Revolve^{®} mis en œuvre dans US 2020/0054824. Le tissu adipeux ainsi purifié présente en particulier un taux de résorption bien inférieur à celui obtenu avec les solutions de purification de l'art antérieur. Les matières polluantes peuvent comprendre l'un au moins de l'huile, du sang ou de l'eau. L'ajout du liquide de lavage permet de récupérer une fraction des matières polluantes du tissu adipeux laquelle fraction est ensuite éliminée par filtration du fait de la centrifugation du contenu du volume de traitement. Comme il sera rappelé plus bas si un tissu adipeux plus pur est souhaité, il est possible de compléter cette séquence d'ajout de liquide de lavage, filtration et évacuation par d'autres traitements permettant d'éliminer une fraction additionnelle des matières polluantes. L'invention utilise une filtration assistée par centrifugation dans laquelle la séparation entre le tissu adipeux et les matières polluantes est facilitée par la mise en rotation des moyens de rétention et de filtration. Le tissu adipeux est soumis à des accélérations centrifuges contre la paroi interne des moyens de rétention et de filtration, permettant de drainer les matières polluantes à travers les pores de ces moyens alors que le tissu adipeux est retenu dans le volume de traitement. La solution selon l'invention se distingue également des techniques de centrifugation en centrifugeuse où le tissu adipeux et les matières polluantes sont présentes dans un contenant totalement fermé et qui mettent en œuvre des accélérations très importantes, généralement supérieures à 400 G, pour obtenir une séparation de phases et peuvent conduire à un endommagement du tissu adipeux. En outre, par rapport à ces techniques, la purification selon l'invention permet d'éliminer l'huile présente dont la réintroduction dans le corps du patient doit être évitée car elle peut former des kystes huileux. Dans un exemple de réalisation, la filtration de la fraction des matières polluantes, contenue dans le liquide de lavage, comprend une succession de phases de mise en rotation des moyens de rétention et de filtration séparées par une ou plusieurs phases d'interruption de cette rotation.

Une telle caractéristique participe avantageusement à améliorer davantage encore la qualité du tissu adipeux purifié obtenu dans la mesure où une mise en rotation intermittente des moyens de rétention et de filtration réduit encore le risque d'endommager le tissu adipeux par rapport à une mise en rotation continue.

Dans un exemple de réalisation, la filtration de la fraction des matières polluantes, contenue dans le liquide de lavage, est effectuée alors que le liquide de lavage est ajouté dans le volume de traitement.

La centrifugation alors que le liquide de lavage est introduit dans le volume de traitement permet de réaliser un brassage de l'ensemble des matériaux présents dans ce volume et éviter tout risque de bouchage des moyens de filtration et de rétention par le tissu adipeux.

Dans un exemple de réalisation, le procédé comprend en outre, après l'évacuation du liquide de lavage, une filtration d'une fraction additionnelle des matières polluantes du tissu adipeux par centrifugation du contenu du volume de traitement par mise en rotation des moyens de rétention et de filtration.

Une telle caractéristique participe avantageusement à améliorer davantage encore la qualité du tissu adipeux purifié obtenu.

En particulier, on peut réaliser après l'évacuation du liquide de lavage :
- un ajout additionnel du liquide de lavage au tissu adipeux,
- la filtration de la fraction additionnelle des matières polluantes, contenue dans le liquide de lavage ainsi ajouté, par centrifugation du contenu du volume de traitement par mise en rotation des moyens de rétention et de filtration, et
- une évacuation du liquide de lavage ainsi ajouté contenant la fraction additionnelle des matières polluantes filtrée.

Une telle caractéristique participe avantageusement à améliorer davantage encore la qualité du tissu adipeux purifié obtenu en permettant l'élimination d'une fraction additionnelle des matières polluantes. Bien entendu et si cela est souhaité, on peut répéter l'ajout du liquide de lavage, la filtration et l'évacuation du liquide de lavage afin d'éliminer une fraction supplémentaire des matières polluantes. Selon l'invention telle que revendiquée, on réalise, avant l'ajout du liquide de lavage, une filtration préliminaire d'une fraction des matières polluantes, présentes dans un liquide interstitiel du tissu adipeux à purifier dans le volume de traitement, par centrifugation du contenu du volume de traitement par mise en rotation des moyens de rétention et de filtration.

Une telle caractéristique participe avantageusement à améliorer davantage encore la qualité du tissu adipeux purifié obtenu en permettant notamment de réduire davantage la quantité de matières polluantes.

La filtration préliminaire peut être réalisée alors que le tissu adipeux à purifier est introduit dans le volume de traitement.

Une telle caractéristique permet avantageusement de pouvoir accueillir un volume plus important de tissu adipeux dans le volume de traitement du fait de l'élimination de l'eau au fur et à mesure de l'introduction du tissu adipeux.

En particulier, la filtration préliminaire peut comprendre une succession de phases de mise en rotation des moyens de rétention et de filtration séparées par une ou plusieurs phases d'interruption de cette rotation.

Une telle caractéristique participe avantageusement à réduire davantage encore la quantité de matières polluantes dans le tissu adipeux purifié obtenu tout en réduisant encore le risque d'endommager le tissu adipeux par rapport à une mise en rotation continue.

Dans un exemple de réalisation, on impose aux matières polluantes une accélération comprise entre 5 G et 40 G lors de la mise en rotation des moyens de rétention et de filtration. Ces valeurs d'accélérations peuvent être imposées aux matières polluantes contenues dans le liquide de lavage et/ou aux matières polluantes contenues dans le liquide interstitiel lors de la filtration préliminaire qui peut être réalisée avant l'ajout du liquide de lavage.

Une telle caractéristique participe avantageusement à optimiser l'élimination des liquides présents dans le tissu adipeux et notamment de l'eau interstitielle et à améliorer davantage encore la qualité du tissu adipeux purifié. Par ailleurs, le fait de limiter l'accélération à des valeurs bien inférieures à celles mises en œuvre en centrifugation classique participe également à obtenir un tissu adipeux de qualité optimale et avec une légère quantité de liquide interstitiel résiduel lui permettant d'être aisément injectable dans le corps du patient.

En particulier, une accélération imposée aux matières polluantes durant la filtration préliminaire est supérieure à une accélération imposée aux matières polluantes durant la filtration des matières polluantes contenues dans le liquide de lavage.

Une telle caractéristique participe avantageusement à réduire davantage encore le taux d'eau interstitielle dans le tissu adipeux purifié obtenu ainsi que de réduire davantage encore la quantité de matières polluantes.

L'invention vise également un tissu adipeux purifié comprenant des cellules adipeuses et présentant une teneur totale en liquides comprise entre 0,05 gramme par millilitre de tissu adipeux et 0,15 gramme par millilitre de tissu adipeux avec une teneur en huile inférieure ou égale à 0,075 gramme par millilitre de tissu.

Un tel tissu adipeux diffère du tissu adipeux directement prélevé chez le patient par sa composition et peut être obtenu par le procédé de purification décrit plus haut. Il présente, en particulier, un taux de résorption limité lorsqu'il est réinjecté dans le corps du patient tout en étant aisément injectable. Le fait de limiter la quantité d'huile dans le tissu permet d'éviter la création de kystes huileux après injection dans le patient qui peuvent représenter un des effets secondaires les plus indésirables des opérations de greffe autologue de tissu adipeux.

Selon un exemple, la teneur totale en liquides est comprise entre 0,07 gramme par millilitre de tissu adipeux et 0,12 gramme par millilitre de tissu adipeux.

Selon un exemple, la teneur en huile dans le tissu adipeux purifié est inférieure ou égale 0,05 gramme par millilitre de tissu adipeux, par exemple inférieure ou égale à 0,03 gramme par millilitre de tissu adipeux. Cette teneur peut par exemple comprise entre 0,005 gramme par millilitre de tissu adipeux et 0,05 gramme par millilitre de tissu adipeux, par exemple entre 0,005 gramme par millilitre de tissu adipeux et 0,03 gramme par millilitre de tissu adipeux.

L'invention vise également un dispositif chirurgical comprenant au moins un réservoir contenant le tissu adipeux purifié tel que décrit plus haut en communication avec un élément d'introduction apte à introduire le tissu adipeux purifié depuis le réservoir dans le corps d'un patient.

Un tel dispositif chirurgical peut correspondre à un dispositif commercialisé sous la référence LipoGrafter^{®} par la société MTFBiologics incorporant le tissu adipeux purifié par le procédé selon l'invention et relié à une canule apte à injecter ce tissu adipeux dans le corps du patient. L'homme du métier reconnaîtra que d'autres dispositifs chirurgicaux sont utilisables pour la réintroduction dans le corps du patient.

### Brève description des dessins

[Fig. 1] La figure 1 est un ordinogramme montrant différentes étapes d'un exemple de procédé selon l'invention.
[Fig. 2] La figure 2 est une vue schématique éclatée d'un exemple de dispositif de purification utilisable dans le cadre de l'invention.
[Fig. 3] La figure 3 est une vue schématique en coupe du dispositif de purification de la figure 2 une fois assemblé.
[Fig. 4] La figure 4 est une vue schématique en coupe montrant le dispositif de purification de la figure 3 après déplacement du fond.
[Fig. 5] La figure 5 représente, de manière schématique, une évolution possible de la vitesse de rotation des moyens de rétention et de filtration utilisable dans le cadre de l'invention pour les étapes de filtration des matières polluantes.
[Fig. 6] La figure 6 représente, de manière schématique et partielle, un dispositif chirurgical comprenant un tissu adipeux purifié selon l'invention et apte à permettre sa réintroduction dans le corps du patient.

### Description des modes de réalisation

La suite s'attache à décrire un exemple de procédé de purification d'un tissu adipeux prélevé du corps d'un patient pour réintroduction dans le corps du patient pour une opération de greffe autologue de tissu adipeux, par exemple pour la chirurgie mammaire ou au niveau d'autres parties du corps.

Le tissu adipeux est prélevé du corps du patient par aspiration à l'aide d'une canule d'aspiration. La canule d'aspiration est reliée à un dispositif de purification, dont un exemple de structure sera décrit ci-dessous, lequel est apte à purifier le tissu adipeux prélevé par filtration des matières polluantes assistée par centrifugation. Le dispositif de purification comprend notamment des moyens de rétention du tissu adipeux et de filtration des matières polluantes, par exemple sous la forme d'une paroi latérale filtrante, ainsi qu'un moyen d'entrainement en rotation apte à mettre ces moyens en rotation. Ces moyens présentent des pores ayant une taille configurée pour laisser passer les matières polluantes, par exemple sous forme d'un milieu liquide, et retenir le tissu adipeux. Durant le prélèvement, le tissu adipeux est introduit dans le dispositif de purification et, plus particulièrement, dans un volume de traitement délimité par les moyens de rétention et de filtration (étape E10).

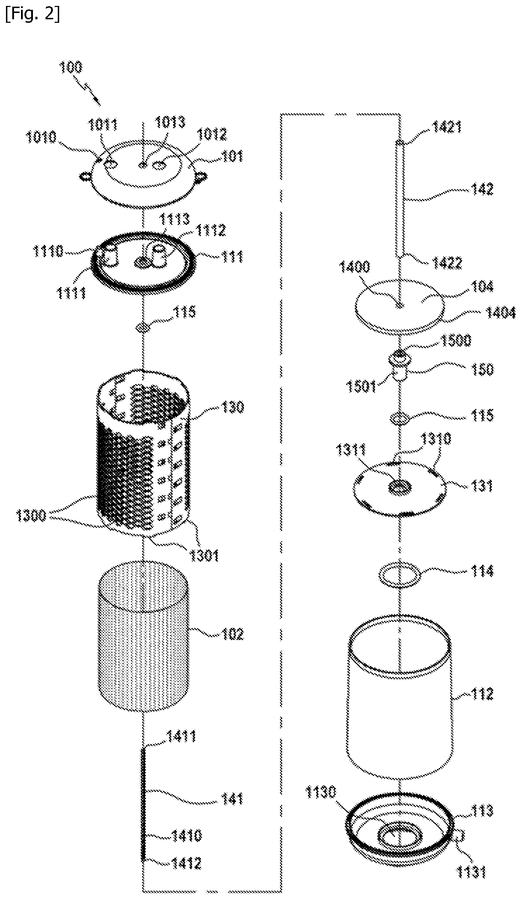

La suite s'attache à décrire, en lien avec les figures 2 à 4, un exemple de dispositif de purification 100 pouvant être mis en œuvre dans le cadre de l'invention. Ce type de dispositif peut être mis en œuvre pour l'ensemble des étapes de filtration des matières polluantes réalisées durant le procédé de purification. Le dispositif 100 est décrit à titre illustratif et non limitatif.

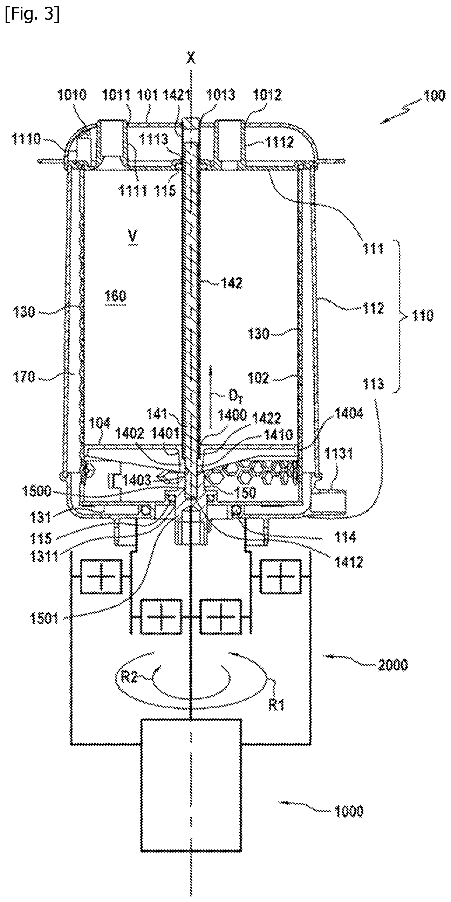

Le dispositif de purification 100 comprend une enceinte étanche 110 formée ici par un capot 111, une paroi latérale 112 et un fond 113. Ces éléments sont fixés ensemble de manière étanche. Les moyens 102 de rétention du tissu adipeux et de filtration des matières polluantes sont présents dans l'enceinte étanche 110. Les moyens 102 délimitent une chambre de centrifugation 160 (visible en figure 3 et qui définit le volume de traitement V destiné à accueillir le tissu adipeux prélevé). Dans l'exemple décrit ici, les moyens 102 présentent une forme cylindrique mais l'homme du métier reconnaîtra que les moyens 102 peuvent présenter d'autres formes adaptées pour la centrifugation. Les moyens 102 présentent une taille de pore configurée pour laisser passer un milieu liquide et retenir un tissu adipeux. La taille de pore est choisie en particulier pour permettre le passage de liquides tels que de l'huile, du sang ou une solution physiologique tout en retenant le tissu adipeux. D'une manière générale, la taille des pores des moyens 102 peut être inférieure ou égale à 1,5 mm, par exemple inférieure ou égale à 0,5 mm. Cette taille peut être comprise entre 0,05 mm et 1,5 mm, par exemple entre 0,2 mm et 0,5 mm. Les moyens 102 peuvent être en matériau polymérique, par exemple en polyester ou en polypropylène mais l'homme du métier reconnaîtra que d'autres matériaux peuvent être utilisés.

Dans l'exemple décrit ici, un élément de rigidification 130 est présent entre la paroi 112 de l'enceinte étanche 110 et les moyens 102. Il a notamment pour fonction d'assurer la tenue structurale des moyens 102 pendant la centrifugation. L'élément de rigidification 130 est par exemple réalisé en matériau métallique ou plastique et présente une structure ajourée définissant une pluralité d'ouvertures 1300 afin de permettre l'évacuation du milieu liquide drainé par les moyens 102.

L'élément de rigidification 130 est associé à un plateau rotatif 131. Plus précisément, l'élément de rigidification 130 comporte à son extrémité inférieure des dents 1301 qui coopèrent avec des rainures 1310 présente au voisinage de la périphérie externe du plateau rotatif 131. L'homme du métier reconnaîtra que l'élément 130 peut être relié au plateau 131 de différentes manières. Le plateau rotatif 131 est relié à un moyen d'entrainement en rotation qui peut être manuel ou motorisé. Dans l'exemple décrit ici, le plateau rotatif est relié à un moteur électrique 1000, comme un moteur pas à pas ou un moteur à courant continu sans balais, via un embrayage bidirectionnel 2000 configuré pour assurer l'entrainement en rotation du plateau rotatif 131 suivant un premier sens de rotation R1 (figure 3).

Des joints 114 et 115 sont placés respectivement en-dessous et au-dessus du plateau rotatif 131 afin d'assurer l'étanchéité dans la partie inférieure du dispositif de purification.

La centrifugation est réalisée par mise en rotation des moyens 102. Plus précisément, le moteur électrique 1000 est commandé suivant le premier sens de rotation R1 pour entrainer le plateau rotatif 131 et l'élément de rigidification 130 en prise avec le plateau 131. La mise en rotation du plateau 131 et de l'élément de rigidification 130 entraine la mise en rotation des moyens 102 qui sont solidaires de l'élément de rigidification 130, en étant par exemple collés ou clipsés à ce dernier. La vitesse du moteur électrique 1000 est contrôlée de manière à appliquer une force centrifuge au matériau dans le volume V. Ainsi, un tissu adipeux présent dans le volume V sera soumis à une force centrifuge contre la paroi interne des moyens 102, ce qui permet de drainer efficacement les matières polluantes du tissu adipeux, contenues dans le liquide de lavage ou le liquide interstitiel selon le stade de la purification, sans abimer le tissu adipeux.

Durant la centrifugation, ces matières polluantes traversant les moyens 102 et l'élément de rigidification 130 sont collectées dans un volume 170 délimité entre l'élément de rigidification 130 et la paroi 112 de l'enceinte 110. On peut ensuite évacuer le liquide de lavage ou le liquide interstitiel contenant les matières polluantes via un port d'évacuation 1131 présent sur le fond 113 de l'enceinte 110. Dans l'exemple décrit ici, le capot 111 comprend trois ports 1110, 1111 et 1112 destinés à être reliés respectivement à un dispositif d'aspiration de tissu adipeux (pompe à vide permettant le prélèvement), à un conduit pour l'introduction du tissu adipeux prélevé dans le volume V et à un dispositif de délivrance du liquide de lavage. Le dispositif de purification comprend un couvercle 101 comportant des ouvertures 1010, 1011 et 1012 qui coopèrent avec les ports 1110, 1111 et 1112 du capot 111. La pompe à vide reliée au dispositif de purification 100 qui permet le prélèvement de tissu adipeux et son introduction dans le volume V peut également être reliée au port d'évacuation 1131. Le tissu adipeux prélevé est retenu dans le dispositif 100 par les moyens 102. La pompe à vide sert à également à évacuer les matières polluantes, contenues dans le liquide de lavage ou le liquide interstitiel, de l'intérieur du dispositif 100 par aspiration. Les matières aspirées sont récupérées dans une poubelle en communication avec la pompe à vide et le port 1131.

Le dispositif de purification peut en outre comprendre un fond 104 du volume V, par exemple sous la forme d'un plateau de collecte mobile. Comme illustré dans l'exemple des figures 2 et 3, le plateau de collecte 104 est mobile en translation dans une direction D_{T} suivant l'axe X des moyens 102. Plus précisément, le dispositif de purification 100 comprend une tige filetée 141 qui s'étend verticalement à l'intérieur de la chambre de centrifugation 160 suivant l'axe X des moyens 102. L'extrémité inférieure 1412 de la tige filetée est reliée au moteur électrique 1000 par l'intermédiaire d'un guide 150 et de l'embrayage bidirectionnel 2000. Le guide 150 comprend un logement 1500 dans lequel l'extrémité inférieure 1412 est fixée. Une portion inférieure 1501 du guide 150 est connectée à une partie de l'embrayage bidirectionnel 2000 qui est en prise avec le moteur électrique 1000 que lorsque celui-ci transmet un mouvement de rotation suivant un deuxième sens de rotation R2 opposé au premier sens de rotation R1 utilisé pour la centrifugation. Lorsque le moteur électrique 10 tourne suivant le premier sens de rotation R1, aucun mouvement de rotation n'est transmis par l'embrayage bidirectionnel 2000 au guide 150 auquel est relié la tige filetée 141 et les moyens 102 sont mis en rotation du fait de la rotation du plateau rotatif 131. On réalise ainsi la filtration des matières polluantes, contenues dans le liquide de lavage ou le liquide interstitiel, au travers des moyens 102. Lorsque le moteur 1000 tourne suivant le deuxième sens de rotation R2, aucun mouvement de rotation n'est transmis par l'embrayage bidirectionnel 2000 au plateau rotatif 131 et, par conséquent, aux moyens 102. Le plateau de collecte 104 comporte sur sa face supérieure une ouverture centrale 1400 prolongée par un col 1401 qui s'étend à partir de la face inférieure du plateau 104. Le col 1401 comprend une portion 1402 comportant un taraudage 1403 qui coopère avec un filetage 1411 de la tige filetée 141. Ainsi, lorsque que la tige filetée est entrainée en rotation suivant le deuxième sens de rotation R2, le plateau de collecte 104 s'élève dans la chambre de centrifugation 160 suivant la direction D_{T}.

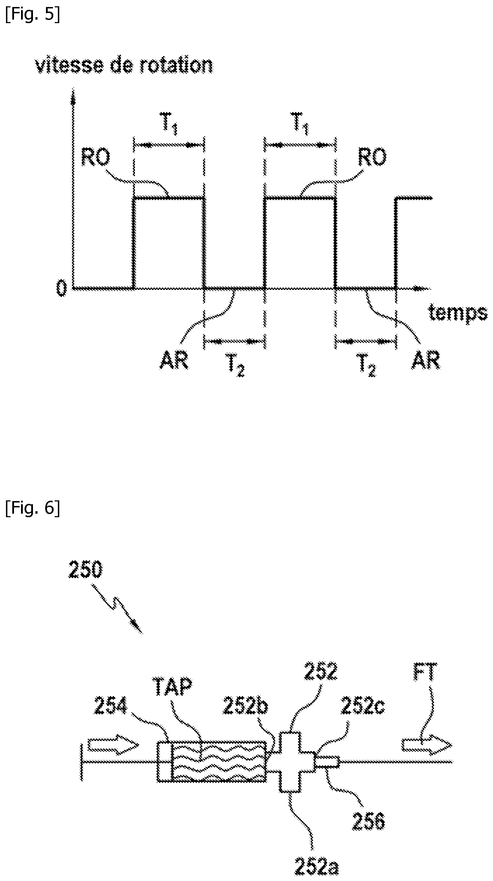

Le bord périphérique 1404 du plateau de collecte 104 est en vis-à-vis de la paroi interne des moyens 102. Ainsi, lorsque le plateau de collecte 104 est déplacé en translation verticale suivant la direction D_{T}, celui-ci joue un rôle de piston qui permet de racler la paroi interne du filtre de manière à collecter davantage de tissu adipeux et faciliter l'évacuation du tissu adipeux purifié en sommet du dispositif pour introduction dans le corps du patient via un dispositif de réintroduction qui sera décrit dans la suite en lien avec la figure 6.

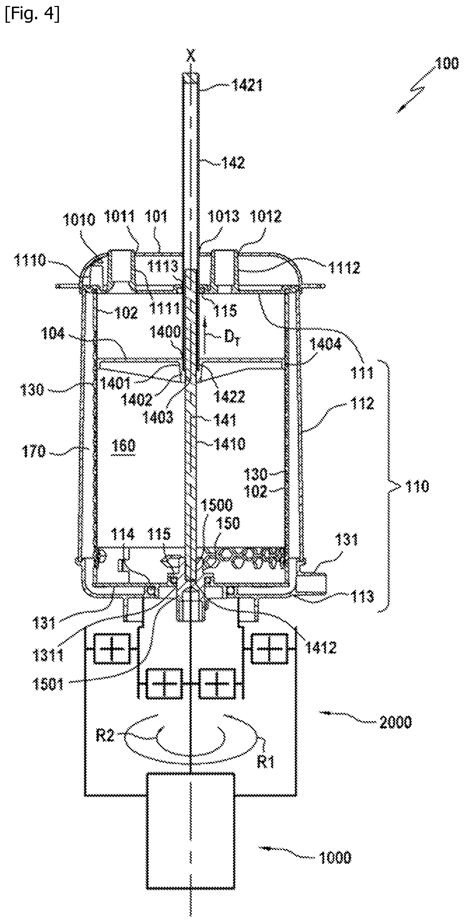

Selon un aspect particulier, la tige filetée 141 peut être logée dans une gaine de protection 142. La gaine de protection 142 qui s'étend entre une extrémité supérieure 1421 et une extrémité inférieure 1422 permet d'éviter à la tige filetée 141 d'entrer en contact avec le tissu adipeux qui en s'accumulant au niveau du taraudage 1403 du plateau de collecte 104 peut bloquer le déplacement de celui-ci. La figure 4 montre le dispositif de purification 100 après actionnement du moteur électrique 1000 dans le deuxième sens de rotation R2 permettant d'entraîner en rotation la tige filetée 141. La rotation de la tige filetée 141 entraîne la translation verticale du plateau de collecte 104 suivant la direction D_{T}.

L'extrémité inférieure 1422 de la gaine de protection est fixée dans l'ouverture 1400 du plateau de collecte 104. Afin de permettre le mouvement de la gaine de protection 142 lors du déplacement du plateau 104, le capot 111 et le couvercle 101 comportent respectivement une ouverture 1113 et une ouverture 1013 au travers desquelles la gaine 142 coulisse. Un joint 115 est présent autour de l'ouverture 1113 afin de préserver l'étanchéité en sommet de la chambre de centrifugation.

Dans un autre exemple de réalisation, les moyens 102 sont en un matériau rigide autoporteur comme par exemple un matériau métallique. Dans ce cas, l'élément de rigidification 130 n'est plus nécessaire et ce sont les moyens 102 autoporteurs qui sont directement en prise avec le plateau rotatif 131.

La suite décrit des détails sur l'étape de filtration d'une fraction des matières polluantes qui sont valables quelle que soit l'étape de filtration considérée, notamment pour la filtration préliminaire (étape E20), ainsi que pour les filtrations des étapes E40, E60 et E90 qui seront décrites plus bas.

La filtration d'une fraction des matières polluantes, contenue dans le liquide de lavage ou dans le liquide interstitiel, est effectuée par centrifugation du contenu du volume V par mise en rotation des moyens 102. Les moyens 102 sont mis en rotation sur eux-mêmes. Les moyens 102 sont mis en rotation autour de l'axe X. L'axe X peut correspondre à l'axe de la hauteur des moyens 102, et par exemple à un axe de symétrie ou de révolution de ces moyens 102. Lorsque les moyens 102 sont mis en rotation, le tissu adipeux et les matières polluantes peuvent subir une accélération supérieure ou égale à 8 G, par exemple supérieure ou égale à 10 G ou supérieure ou égale à 12G. Cette accélération mesurée en G correspond au rapport entre l'accélération subie par le matériau et l'accélération de la pesanteur terrestre, laquelle est d'environ 9,81 m²/s. L'accélération subie par le matériau correspond au rapport de la force centrifuge appliquée et de la masse du matériau considéré. La force centrifuge appliquée est égale à m*ω² * R où m est la masse de l'objet considéré, ω est la vitesse angulaire des moyens 102 exprimée en rad/s et R la distance de l'axe X de rotation au centre de gravité de l'objet considéré. Lors de la rotation des moyens 102, le tissu adipeux et les matières polluantes peuvent subir une accélération inférieure ou égale à 40 G, par exemple inférieure ou égale à 30 G, voire inférieure ou égale à 25 G ou inférieure ou égale à 20 G. Cette accélération peut être comprise entre 8 G et 40 G ou entre 8 G et 30 G ou entre 8 G et 25 G ou entre 8 G et 20 G. Cette accélération peut être comprise entre 10 G et 40 G ou entre 10 G et 30 G ou entre 10 G et 25 G ou entre 10 G et 20 G. Cette accélération peut être comprise entre 12 G et 40 G ou entre 12 G et 30 G ou entre 12 G et 25 G ou entre 12 G et 20 G. La durée d'une étape de filtration peut typiquement être supérieure ou égale à 5 secondes. Cette durée peut être inférieure ou égale à 60 secondes. Cette durée peut par exemple comprise entre 5 secondes et 60 secondes, par exemple entre 15 secondes et 45 secondes. Quelle que soit l'étape de filtration considérée, les moyens 102 peuvent être mis en rotation de manière intermittente, c'est-à-dire en imposant successivement au moins une première phase RO de mise en rotation des moyens 102, une phase AR d'interruption de cette rotation où les moyens 102 sont immobilisés, et une deuxième phase RO de mise en rotation des moyens 102, comme illustré à la figure 5. L'accélération imposée aux matières polluantes durant la première phase RO de mise en rotation peut être identique ou différente à celle imposée durant la deuxième phase RO de mise en rotation. Ces accélérations peuvent avoir les valeurs en G décrites plus haut. Les phases RO de mise en rotation des moyens 102 peuvent, selon un exemple, avoir une durée T1 supérieure ou égale à 5 secondes, par exemple comprise entre 5 secondes et 15 secondes et les phases d'arrêt AR peuvent, selon un exemple, avoir une durée T2 supérieure ou égale à 2 secondes, par exemple comprise entre 2 secondes et 10 secondes. On a représenté à la figure 5 des phases RO de rotation qui ont des durées T1 identiques, ainsi que des phases d'arrêt AR qui ont également la même durée T2 mais on ne sort pas du cadre de l'invention lorsque ce n'est pas le cas. On ne sort pas du cadre de l'invention si davantage de phases RO de mise en rotation sont effectuées, ainsi on peut réaliser une deuxième phase AR d'interruption de la rotation où les moyens 102 sont immobilisés après la deuxième phase RO de mise en rotation, puis une troisième phase de mise en rotation des moyens 102 et ainsi de suite. On peut ainsi réaliser une alternance de phases de mise en rotation des moyens 102 et de phases d'interruption de cette rotation durant une étape de filtration d'une fraction des matières polluantes. On notera que l'emploi d'une rotation intermittente est avantageux notamment pour la filtration préliminaire (étape E20) et la filtration de la (des) fractions des matières polluantes présente(s) dans le liquide de lavage (étape E60) mais l'on ne sort pas du cadre de l'invention si tout ou partie des étapes de filtration sont effectuées par une mise en œuvre d'une seule phase de mise en rotation des moyens 102 appliquée en continu.

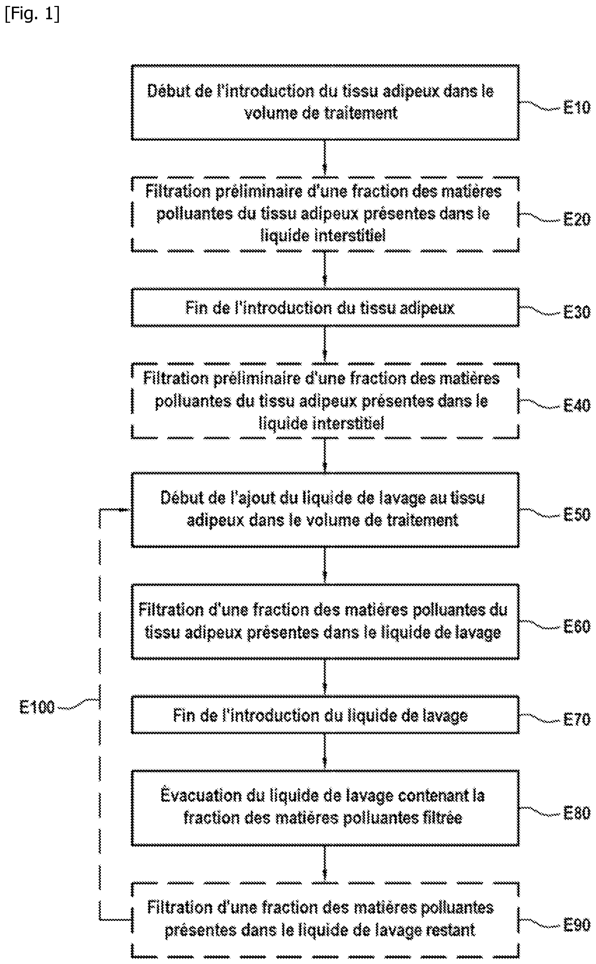

L'étape E20 de la figure 1 concerne la réalisation d'une filtration préliminaire d'une fraction des matières polluantes contenue dans le liquide interstitiel du tissu adipeux en cours d'introduction dans le volume V de traitement. Une rotation intermittente des moyens 102 telle que décrite plus haut peut être imposée durant cette étape. La filtration préliminaire permet d'éliminer des matières polluantes sous forme liquide présentes dans le tissu adipeux introduit, comme du sang, de l'eau ou de l'huile. Durant cette phase, il n'y a pas d'introduction d'un liquide de lavage dans le volume V, seul le tissu adipeux prélevé est introduit dans le volume V. Ainsi, on peut initier le prélèvement du tissu adipeux (étape E10) puis lorsqu'un premier volume prédéterminé a été introduit dans le volume V réaliser une première phase RO de mise en rotation des moyens 102 alors que le prélèvement du tissu adipeux continue aboutissant à l'introduction de davantage de tissu adipeux dans le volume V. Selon un exemple, la première phase RO de mise en rotation des moyens 102 peut être initiée lorsqu'un volume compris entre 50 cm³ et 250 cm³ de tissu adipeux a été introduit dans le volume V. Les moyens 102 sont ensuite arrêtés (phase AR) alors que le prélèvement de tissu adipeux continue lors de cette phase AR d'interruption après la première phase de mise en rotation. Lorsqu'un deuxième volume prédéterminé de tissu adipeux a été introduit dans le volume V, le deuxième volume prédéterminé étant supérieur au premier volume prédéterminé, on peut initier la deuxième phase RO de mise en rotation des moyens 102, et ainsi de suite si l'on souhaite poursuivre la filtration préliminaire. L'exemple de la figure 1 concerne le cas où la filtration préliminaire (étape E20) est effectuée alors que le tissu adipeux est introduit dans le volume V mais l'on ne sort pas du cadre de l'invention lorsque la filtration préliminaire est initiée seulement après achèvement du prélèvement du tissu adipeux.

Une fois la quantité souhaitée de tissu adipeux introduit dans le volume V, le prélèvement est arrêté (étape E30, arrêt de l'introduction de tissu adipeux dans le volume V). On peut alors mettre à nouveau en rotation les moyens 102 de sorte à filtrer une fraction des matières polluantes contenue dans le liquide interstitiel (étape E40, optionnelle), avant l'ajout du liquide de lavage (étape E50) et alors que du tissu adipeux n'est plus introduit dans le volume V. Une seule mise en rotation continue des moyens 102 peut être effectuée durant l'étape E40, mais l'on pourrait, en variante, appliquer une rotation intermittente, comme décrit plus haut.

La pompe à vide ayant servi au prélèvement est ensuite activée de sorte à évacuer par aspiration le liquide interstitiel contenant les matières polluantes filtré. La durée de l'évacuation peut être supérieure ou égale à 5 secondes, par exemple comprise entre 5 secondes et 60 secondes.

Le liquide de lavage est ensuite introduit dans le volume V (étape E50). Le liquide de lavage se mélange avec le tissu adipeux prélevé afin de récupérer une fraction des matières polluantes de ce tissu. Le liquide de lavage peut ainsi récupérer du sang, de l'eau et de l'huile présents dans le tissu adipeux. Le liquide de lavage peut être une solution physiologique. Le liquide de lavage est introduit au travers du port 1112 dans l'exemple de dispositif 100 décrit plus haut. On notera que le liquide de lavage peut être ajouté alors que les moyens 102 sont mis en rotation comme décrit plus haut et en particulier avec une rotation intermittente. Les phases de mise en rotation et d'interruption(s) de la rotation peuvent être réalisées durant l'introduction du liquide de lavage (alors qu'il continue d'être introduit dans le volume V). L'ajout du liquide de lavage peut être effectué simultanément à la filtration de la fraction des matières polluantes contenue dans le liquide de lavage (étape E60). Lors de cette étape E60, le liquide de lavage contenant la fraction des matières polluantes traverse les pores des moyens 102 pour sortir du volume V dans lequel le tissu adipeux est retenu. Les matières polluantes sont récupérées dans le volume 170 dans l'exemple de dispositif 100 illustré. On réalise ainsi une séparation du tissu adipeux et du liquide de lavage contenant la fraction des matières polluantes. On notera que l'on ne sort pas du cadre de l'invention si l'ajout du liquide de lavage et la filtration (étape E60) sont effectuées de manière séquentielle, la filtration (étape E60) étant initiée une fois l'ajout du liquide de lavage terminé (étape E70).

Une fois l'introduction du liquide de lavage achevée (étape E70), on réalise une aspiration par la pompe à vide au travers du port 1131 afin d'évacuer le liquide de lavage contenant la fraction des matières polluantes filtrée du dispositif 100 et le récupérer dans une poubelle (étape E80). Cette aspiration peut par exemple être réalisée à l'aide de la pompe à vide ayant servi au prélèvement du tissu adipeux. L'évacuation peut être réalisée pendant une durée supérieure ou égale à 5 secondes, par exemple comprise entre 5 secondes et 60 secondes. Si cela est souhaité, on peut ensuite continuer à éliminer le liquide de lavage restant contenant des matières polluantes en effectuant une mise en rotation des moyens 102 suivie d'une aspiration de manière similaire à ce qui a été décrit plus haut (étape E90 optionnelle) sans ajout de liquide de lavage durant cette étape. On peut ensuite, si cela est souhaité, répéter les étapes d'ajout de liquide de lavage (étape E50), de filtration d'une fraction des matières polluantes présentes dans le liquide de lavage (étape E60) et d'évacuation du liquide de lavage (étape E80), comme matérialisé par la flèche E100 (optionnelle).

La purification peut également être complétée après les séquences décrites plus haut par un compactage du tissu adipeux dans lequel le fond 104 peut être remonté, tel un piston (voir position du fond 104 en figure 4), et une aspiration peut être réalisée sur le tissu adipeux compacté pour évacuer l'air et des matières polluantes éventuellement encore présentes.

Les inventeurs ont réalisé des essais visant à mesurer la diminution de la quantité d'huile et de liquides dans le tissu adipeux purifié par rapport au tissu adipeux directement prélevé dans le corps du patient. Les résultats obtenus sur cinq séries d'essais sont indiqués dans les tableaux 1 à 5 ci-dessous.

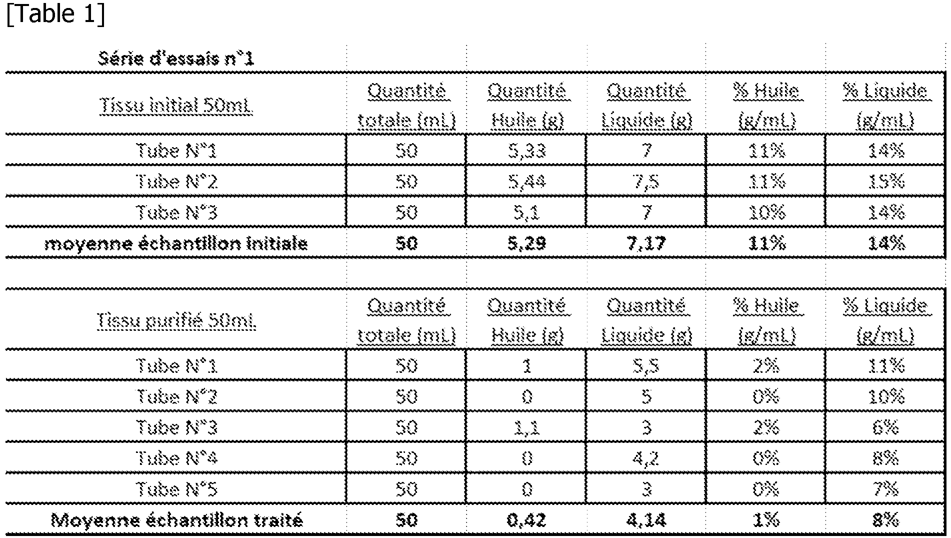

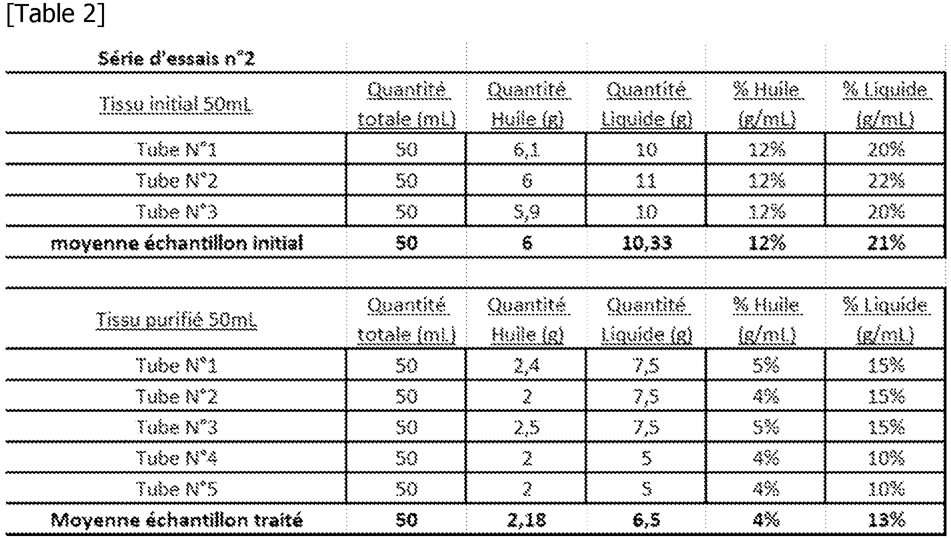

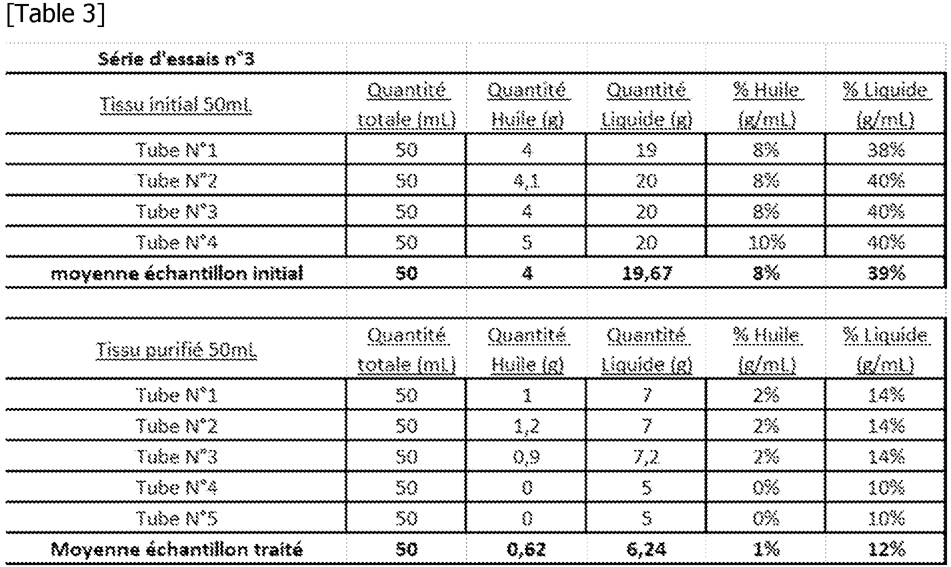

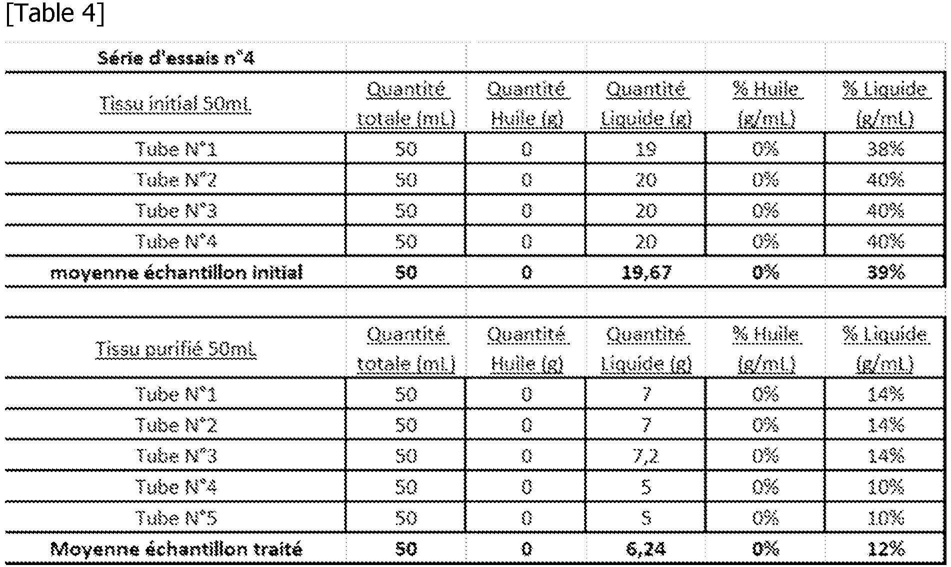

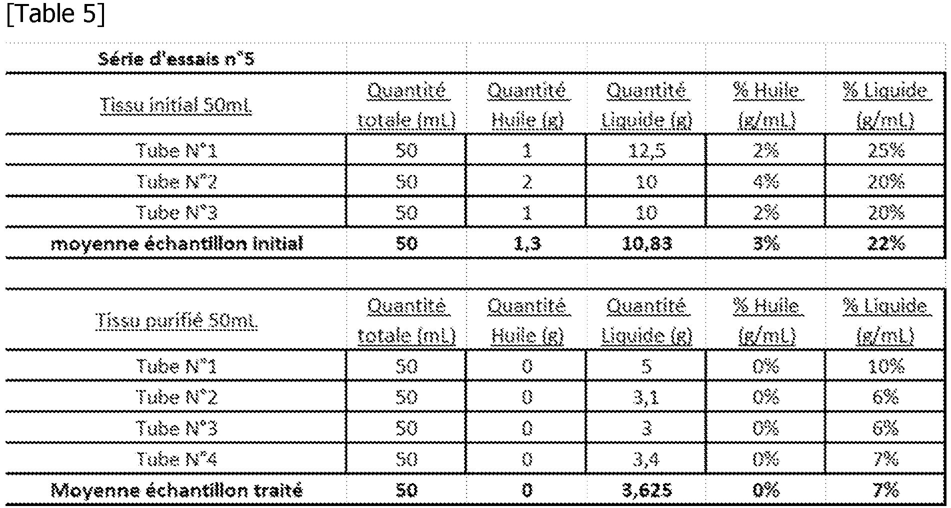

Les moyennes sur les cinq séries d'essais réalisés sont fournies dans le tableau 6 ci-dessous.

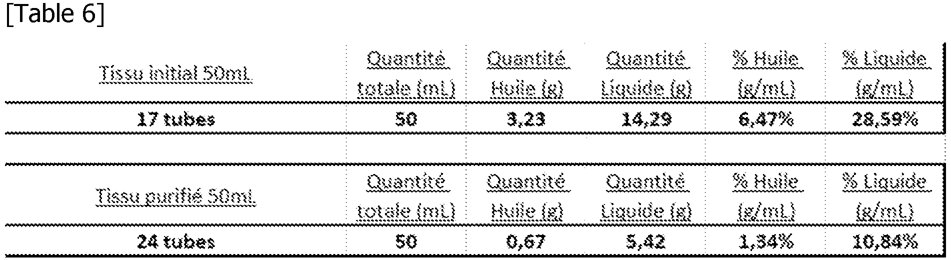

La méthode de mesure employée pour quantifier les quantités de liquide et d'huile sont fournies ci-dessous.

3 à 5 x 50mL de tissu adipeux sont centrifugés à 1600 G (3000 tours/minute) dans des tubes gradués de 50mL pendant 3 minutes. A la sortie les tubes présentent 3 phases (de bas en haut) :
- la phase liquide (sang et liquide d'infiltration ou liquide de lavage),
- la phase de tissu adipeux, et
- la phase d'huile.

Pour chaque tube l'huile est aspirée délicatement avec une micropipette et un cône de 1000µL. L'huile est déposée dans une coupelle plastique pour être pesée sur une balance de précision (0,001g). Pour chaque tube, une pipette de 10mL reliée à un pipeteur électrique est enfoncée délicatement dans le fond de chaque tube. Tout le liquide est aspiré ne laissant dans le tube que la phase de tissu adipeux. Pour chaque tube le liquide est transféré dans un réceptacle puis pesé sur une balance de précision (0,001g).

Une fois la purification terminée, le chirurgien réalise la réintroduction de ce tissu dans le corps du patient. Dans l'exemple de dispositif 100 illustré aux figures 2 à 4, le tissu adipeux purifié peut être compacté contre le capot 111 dans la configuration du dispositif de la figure 4 et introduit dans un dispositif chirurgical, par exemple tel qu'un un dispositif commercialisé sous la référence LipoGrafter^{®} par la société MTFBiologics, afin de réaliser la réintroduction dans le corps du patient. Le dispositif d'introduction peut par exemple être relié au port 1112 de l'exemple de dispositif 100 décrit plus haut et la pression de compaction appliquée par le fond 104 au tissu adipeux purifié peut être suffisante pour permettre le transfert du tissu vers le dispositif de réintroduction. En variante, on peut disposer une pompe entre le volume de traitement V et le dispositif de réintroduction afin de provoquer ce transfert. On a représenté à la figure 6 de manière schématique et partielle un exemple de dispositif 250 apte à permettre la réintroduction du tissu adipeux purifié TAP dans le corps du patient. Le dispositif 250 comprend un corps 252 muni d'un premier port 252a relié au dispositif 100 (liaison non représentée), un deuxième port 252b relié à une seringue 254 et un troisième port 252c relié à une canule d'injection 256. Le tissu adipeux purifié est introduit depuis le volume de traitement V dans la seringue 254 au travers du premier port 252a. Le premier port 252a est muni d'un clapet anti-retour de façon à permettre l'injection du tissu adipeux purifié TAP dans le patient au travers de la canule 256 par pression du piston de la seringue 254. Le chirurgien peut agir directement sur la seringue 254 afin de provoquer l'introduction de matière dans le corps du patient, ou en variante cette action peut être automatisée.

L'expression « comprise entre ... et ... » doit se comprendre comme incluant les bornes.

## Revendications

1. Procédé de purification d'un tissu adipeux, comprenant au moins :
- l'ajout d'un liquide de lavage (E50) à un tissu adipeux à purifier comprenant des matières polluantes et présent dans un volume (V) de traitement délimité par des moyens (102) de rétention du tissu adipeux et de filtration des matières polluantes,
- la filtration (E60) d'une fraction des matières polluantes du tissu adipeux, contenue dans le liquide de lavage, par centrifugation du contenu du volume de traitement par mise en rotation des moyens de rétention et de filtration, et
- une évacuation (E80) du liquide de lavage contenant la fraction des matières polluantes filtrée,
**caractérisé en ce que** dans ce procédé on réalise, avant l'ajout du liquide de lavage, une filtration préliminaire (E20) d'une fraction des matières polluantes, présentes dans un liquide interstitiel du tissu adipeux à purifier dans le volume de traitement, par centrifugation du contenu du volume de traitement par mise en rotation des moyens de rétention et de filtration.

2. Procédé selon la revendication 1, dans lequel la filtration (E60) de la fraction des matières polluantes, contenue dans le liquide de lavage, comprend une succession de phases de mise en rotation des moyens (102) de rétention et de filtration séparées par une ou plusieurs phases d'interruption de cette rotation.

3. Procédé selon l'une quelconque des revendications 1 ou 2, dans lequel la filtration (E60) de la fraction des matières polluantes, contenue dans le liquide de lavage, est effectuée alors que le liquide de lavage est ajouté (E50) dans le volume de traitement (V).

4. Procédé selon l'une quelconque des revendications 1 à 3, dans lequel le procédé comprend en outre, après l'évacuation (E80) du liquide de lavage, une filtration (E90 ; E100) d'une fraction additionnelle des matières polluantes du tissu adipeux par centrifugation du contenu du volume de traitement par mise en rotation des moyens (102) de rétention et de filtration.

5. Procédé selon la revendication 4, dans lequel on réalise après l'évacuation (E80) du liquide de lavage :
- un ajout additionnel (E100+E50) du liquide de lavage au tissu adipeux,
- la filtration (E100+E60) de la fraction additionnelle des matières polluantes, contenue dans le liquide de lavage ainsi ajouté, par centrifugation du contenu du volume de traitement par mise en rotation des moyens (102) de rétention et de filtration, et
- une évacuation (E100+E80) du liquide de lavage ainsi ajouté contenant la fraction additionnelle des matières polluantes filtrée.

6. Procédé selon l'une quelconque des revendications 1 à 5, dans lequel la filtration préliminaire (E20) comprend une succession de phases de mise en rotation des moyens (102) de rétention et de filtration séparées par une ou plusieurs phases d'interruption de cette rotation.

7. Procédé selon l'une quelconque des revendications 1 à 6, dans lequel on impose aux matières polluantes une accélération comprise entre 5 G et 40 G lors de la mise en rotation des moyens (102) de rétention et de filtration.

8. Procédé selon l'une quelconque des revendications 1 à 7, dans lequel une accélération imposée aux matières polluantes durant la filtration préliminaire (E20) est supérieure à une accélération imposée aux matières polluantes durant la filtration (E60) des matières polluantes contenues dans le liquide de lavage.

## Patentansprüche

1. Verfahren zum Reinigen eines Fettgewebes, mindestens umfassend:
- Hinzufügen einer Waschflüssigkeit (E50) zu einem zu reinigenden Fettgewebe, umfassend verunreinigende Stoffe und das in einem Behandlungsvolumen (V) vorhanden ist, das durch Einrichtungen (102) zum Zurückhalten des Fettgewebes und zum Filtern der verunreinigenden Stoffe begrenzt ist,
- Filtern (E60) eines Anteils an verunreinigenden Stoffen des Fettgewebes, die in der Waschflüssigkeit enthalten sind, durch Zentrifugieren des Inhalts des Behandlungsvolumens, indem die Rückhalte- und Filtrationseinrichtungen in Drehung versetzt werden, und
- Ableiten (E80) der Waschflüssigkeit, die den gefilterten Anteil an verunreinigenden Stoffen enthält,
**dadurch gekennzeichnet, dass** bei diesem Verfahren vor Hinzufügen der Waschflüssigkeit eine Vorfiltration (E20) eines Anteils an verunreinigenden Stoffen, die in einer interstitiellen Flüssigkeit des zu reinigenden Fettgewebes in dem Behandlungsvolumen vorhanden sind, durch Zentrifugieren des Inhalts des Behandlungsvolumens durchgeführt wird, indem die Rückhalte- und Filtrationseinrichtungen in Drehung versetzt werden.

2. Verfahren nach Anspruch 1, wobei die Filtration (E60) des Anteils an verunreinigenden Stoffen, die in der Waschflüssigkeit enthalten sind, eine Abfolge von Phasen umfasst, in denen die Rückhalte- und Filtrationseinrichtungen (102) in Drehung versetzt werden, getrennt durch eine oder mehrere Unterbrechungsphasen dieser Drehung.

3. Verfahren nach einem der Ansprüche 1 oder 2, wobei die Filtration (E60) des Anteils an verunreinigenden Stoffen, die in der Waschflüssigkeit enthalten sind, durchgeführt wird, während die Waschflüssigkeit zu dem Behandlungsvolumen (V) hinzugefügt wird (E50).

4. Verfahren nach einem der Ansprüche 1 bis 3, wobei das Verfahren nach dem Ableiten (E80) der Waschflüssigkeit ferner eine Filtration (E90; E100) eines zusätzlichen Anteils an verunreinigenden Stoffen aus dem Fettgewebe durch Zentrifugieren des Inhalts des Behandlungsvolumens umfasst, indem die Rückhalte- und Filtrationseinrichtungen (102) in Drehung versetzt werden.

5. Verfahren nach Anspruch 4, wobei nach dem Ableiten (E80) der Waschflüssigkeit Folgendes durchgeführt wird:
- ein zusätzliches Hinzufügen (E100+E50) der Waschflüssigkeit zu dem Fettgewebe,
- Filtern (E100+E60) des zusätzlichen Anteils an verunreinigenden Stoffen, die in der somit hinzugefügten Waschflüssigkeit enthalten sind, durch Zentrifugieren des Inhalts des Behandlungsvolumens, indem die Rückhalte- und Filtrationseinrichtungen (102) in Drehung versetzt werden, und
- Ableiten (E100+E80) der somit hinzugefügten Waschflüssigkeit, die den zusätzlichen Anteil an gefilterten verunreinigenden Stoffen enthält.

6. Verfahren nach einem der Ansprüche 1 bis 5, wobei die Vorfiltration (E20) eine Abfolge von Phasen umfasst, in denen die Rückhalte- und Filtrationseinrichtungen (102) in Drehung versetzt werden, getrennt durch eine oder mehrere Unterbrechungsphasen dieser Drehung.

7. Verfahren nach einem der Ansprüche 1 bis 6, wobei den verunreinigenden Stoffen eine Beschleunigung zwischen 5 G und 40 G auferlegt wird, wenn die Rückhalte- und Filtrationseinrichtungen (102) in Drehung versetzt werden.

8. Verfahren nach einem der Ansprüche 1 bis 7, wobei eine Beschleunigung, die den verunreinigenden Stoffen während der Vorfiltration (E20) auferlegt wird, größer ist als eine Beschleunigung, die den verunreinigenden Stoffen während der Filtration (E60) der in der Waschflüssigkeit enthaltenen verunreinigenden Stoffen auferlegt wird.

## Claims

1. A method for purifying adipose tissue, comprising at least:
- addition (E50) of a washing liquid to an adipose tissue to be purified comprising contaminants and present in a treatment volume (V) delimited by means (102) of retention of the adipose tissue and filtration of the contaminants,
- filtration (E60) of a fraction of the contaminants of the adipose tissue contained in the washing liquid by centrifuging the contents of the treatment volume by rotating the retaining and filtering means, and
- a discharge (E80) of the washing liquid containing the filtered fraction of contaminants,
**characterized in that** in this method a preliminary filtration (E20) of a fraction of the contaminants present in an interstitial liquid of the adipose tissue to be purified in the treatment volume is carried out before the addition of the washing liquid, by centrifuging the contents of the treatment volume by rotating the retaining and filtering means.

2. The method according to claim 1 wherein the filtration (E60) of the fraction of contaminants contained in the washing liquid comprises a succession of phases of rotation of the retaining and filtering means (102) separated by one or more phases of interruption of this rotation.

3. The method according to one of claims 1 or 2, wherein the filtration (E60) of the fraction of contaminants contained in the washing liquid is carried out while the washing liquid is added (E50) to the treatment volume (V).

4. The method according to any one of claims 1 to 3, wherein the method further comprises, after discharge (E80) of the washing liquid, filtration (E90; E100) of an additional fraction of the contaminants of the adipose tissue by centrifuging the contents of the treatment volume by rotating the retaining and filtering means (102).

5. The method according to claim 4, wherein the following steps are carried out after the discharge (E80) of the washing liquid:
- an additional addition (E100+E50) of the washing liquid to the adipose tissue,
- filtration (E100+E60) of the additional fraction of the contaminants contained in the washing liquid thus added by centrifuging the contents of the treatment volume by rotating the retaining and filtering means (102), and
- a discharge (E100+E80) of the washing liquid thus added containing the additional fraction of the contaminants filtered.

6. The method according to any one of claims 1 to 5, wherein the preliminary filtration (E20) comprises a succession of phases of rotation of the retaining and filtering means (102) separated by one or more phases of interruption of this rotation.

7. The method according to any one of claims 1 to 6, wherein the contaminants are accelerated to between 5 G and 40 G when the retaining and filtering means (102) are rotated.

8. The method according to any one of claims 1 to 7, wherein an acceleration imposed on the pollutants during the preliminary filtration (E20) is greater than an acceleration imposed on the pollutants during the filtration (E60) of the pollutants contained in the washing liquid.
